# FASCICULE DE BREVET EUROPEEN

(11) **EP 1 986 583 B1**
(45) Date de publication et mention de la délivrance du brevet: **16.12.2009**
(21) Numéro de dépôt: 07730926.8
(22) Date de dépôt: 06.02.2007
(51) Int. Cl.: A61F 13/62, D04H 3/10

(54) **FERMETURE AUTO-AGRIPPANTE EN NON-TISSE POUR UN VETEMENT**
VLIESKLETTVERSCHLUSS FÜR KLEIDUNG
NONWOVEN HOOK-AND-LOOP FASTENER FOR GARMENTS

(30) Priorité: 17.02.2006 FR 0601394
(43) Date de publication de la demande: 05.11.2008
(73) Titulaire: Rieter Perfojet, 38330 Montbonnot (FR)
(72) Inventeur: NOELLE, Frédéric, F-38330 St Nazaire Les Eymes (FR); ROUSSEL, Sophie, F-69570 Dardilly (FR)
(74) Mandataire: Eidelsberg, Victor Albert
(86) Numéro de dépôt international: PCT/FR2007/000210
(87) Numéro de publication internationale: WO 2007/093685

(56) Documents cités:
- EP-A- 0 937 420
- EP-A- 1 469 105
- WO-A-97/19808
- WO-A-03/060215
- WO-A-03/105621
- WO-A-2004/038081
- US-A- 4 840 829
- US-B1- 6 903 034

## Description

La présente invention se rapporte aux fermetures auto-agrippantes et aux vêtements qui se ferment par des fermetures de ce genre.

Les systèmes de fermeture à bandes du type crochets et boucles sont largement employés dans différents domaines d'application comme par exemple l'hygiène, les vêtements, l'automobile. Ils sont composés d'une partie mâle comportant des crochets et d'une partie femelle comportant des boucles dans lesquelles viennent s'accrocher les crochets de la partie mâle. Comme la partie femelle est souvent réalisée en un matériau souple, elle est généralement contre-collée ou laminée à un support plus rigide.

Dans le secteur de l'hygiène, pour des produits jetables à usage limité dans le temps, il y a un besoin pour des zones d'accroche de prix faible, moins chers que des produits textiles tricotés ou tissés. Les couches bébé et leurs équivalents pour l'incontinence adulte sont des exemples d'applications nécessitant des zones d'accroche de prix modique, pouvant être ouvert et fermé un nombre restreint de fois, typiquement 3 fois avant d'être jetés. Les' zones d'accroche pour l'hygiène doivent aussi être très douces au toucher, et dans certains cas comme les couches bébé pouvoir être imprimables sur leur envers dans un but décoratif.

Or on a trouvé et c'est ce qui fait l'objet de la présente invention, une structure de non-tissé qui est particulièrement bien adaptée à la réalisation de zones d'accroche ou partie femelle pour des applications dans lesquelles le nombre d'ouverture et de fermetures du système auto agrippant est limité comme par exemple dans le domaine de l'hygiène.

L'invention a donc pour objet une utilisation suivant l'une des revendications 1 à 7.

L'invention a donc pour objet l'utilisation, comme partie femelle d'une fermeture auto-agrippante, d'un non-tissé
- qui est constitué de filaments,
- dont la cohésion n'est donnée que par l'enchevêtrement des filaments,
- qui a une épaisseur comprise entre 0,1 et 2,0 mm, de préférence entre 0,25 mm et 1,20 mm,
- qui a un grammage compris entre 10 et 100 g/m2, de préférence entre 15 et 40 g/m2 et
- qui a des ajours en un nombre de 3 à 80 par cm2 , de préférence entre 4 et 40 par cm2, sur les parois latérales desquels les filaments enchevêtrés sont intacts.

Les parties femelles de fermetures selon l'invention présentent une excellente accroche avec la plupart des crochets connus. Il est aussi possible de repositionner plusieurs fois les crochets au même endroit tout en conservant de bonnes caractéristiques de résistance. Le toucher est textile et doux et convient particulièrement bien aux attentes des consommateurs de produits d'hygiène comme les couches pour bébé. Dans le cas des utilisations dans des produits pour l'hygiène, les non-tissés selon l'invention présentent aussi l'avantage par rapport à des produits commerciaux de ne pas nécessiter de laminage ou contre collage avec un film imprimé. En effet l'impression peut être réalisée directement sur l'envers du non-tissé par des techniques connues comme la flexographie. Et un autre avantage des non-tissés salon l'invention est qu'ils sont peu chers, moins chers que les textiles à boucles comme les tricots ou autres produits apparentés aux textiles.

L'invention a pour objet l'utilisation, comme partie femelle d'une fermeture auto-agrippante, d'un non-tissé constitué de filaments continus de matières thermoplastiques comme par exemple le PP, PE, PET, PA, PLA... Les filaments ont notamment un diamètre compris entre 5 et 50 microns et de préférence compris entre 10 et 30 microns. Les filaments sont disposés en un réseau complexe en 3 dimensions ayant la caractéristique de ne présenter aucun point de fusion ou de collage des filaments les uns aux autres. En d'autres termes, la cohésion des filaments est assurée par leur unique enchevêtrement, ce qui permet d'obtenir une efficacité maximale d'accrochage des crochets dans le réseau fibreux. La majorité de la longueur des filaments est utilisable, ce qui en fait une zone d'accroche particulièrement efficace. La présence d'ajours en améliore l'efficacité. Ces ajours s'étendent d'une face vers l'autre face. Ils peuvent être de forme géométrique carrée, rectangulaire, circulaire ou elliptique. Ils se terminent par une ouverture à la face opposée. Les ajours sont au nombre de 3 à 80 par cm2 et de préférence de 4 à 40 par cm2. Les ajours sont délimités et séparés les uns des autres par le réseau fibreux. Ils sont de préférence ordonnés en quinconce ou alignés, mais peuvent aussi être disposées de manière aléatoire.

En général, ces ajours ne sont pas parfaitement cylindriques mais plutôt de forme conique. L'angle du cône est compris entre 5 et 45° observé au microscope optique de grossissement 20 fois. Les parois des cônes sont constituées des ajours de filaments enchevêtrés non coupés, non cassés. Les filaments sont intacts sur les parois latérales.

A la différence de perforations qui affaibliraient le non-tissé, ces ajours obtenus sans enlèvement de matière et par repoussement de la matière dans les intervalles entre les ajours, renforcent le pouvoir d'accrochage des crochets dans la partie femelle en augmentant la quantité de matière dans les intervalles sans pour autant augmenter le grammage moyen. La partie femelle est rendue plus efficace sans être plus coûteuse.

Ces ajours ont au moins deux dimensions principales dans le plan du non-tissé que l'on peut nommer hauteur et largeur.

La plus petite dimension et la plus grande dimension sont comprises entre 0,3 et 3,5 mm et de préférence entre 0,5 et 3,0 mm. Dans certains cas particulier la plus petite et la plus grande dimension sont identiques. C'est par exemple le cas du carré.

Le pas des ajours, défini par la distance d'axe médian à axe médian de deux ajours consécutifs est habituellement compris entre 1.2 fois et 3.0 fois la plus petite dimension des ajours et de préférence compris entre 1.3 fois et 2.5 fois la plus petite dimension des ajours.

On obtient le plus facilement les ajours lorsque la plus grande dimension des ajours est au moins égale à l'épaisseur du non-tissé et au plus égale à 3 fois cette épaisseur.

La force de cisaillement donnée par la partie femelle lors de tests avec crochets est au minimum de 10 N et de préférence au minimum de 13 N.

La force de pelage lors de tests avec crochets est au minimum de 10 N et de préférence au minimum de 15 N.

Les non-tissés utilisés selon l'invention sont transparents et imprimables sur leur envers et l'impression est visible sur l'endroit ce qui constitue un avantage non négligeable par rapport aux non-tissés commercialisés actuellement qui eux sont difficilement imprimables et dont la transparence est moins bonne que les non-tissés selon l'invention.

Les non-tissés utilisés selon l'invention sont produits sur une machine commercialisée par la société Rieter Perfojet sous la marque commerciale Spunjet. La formation de la nappe de filaments est réalisée par une tour spunbond et sa consolidation par le procédé de liage hydraulique.

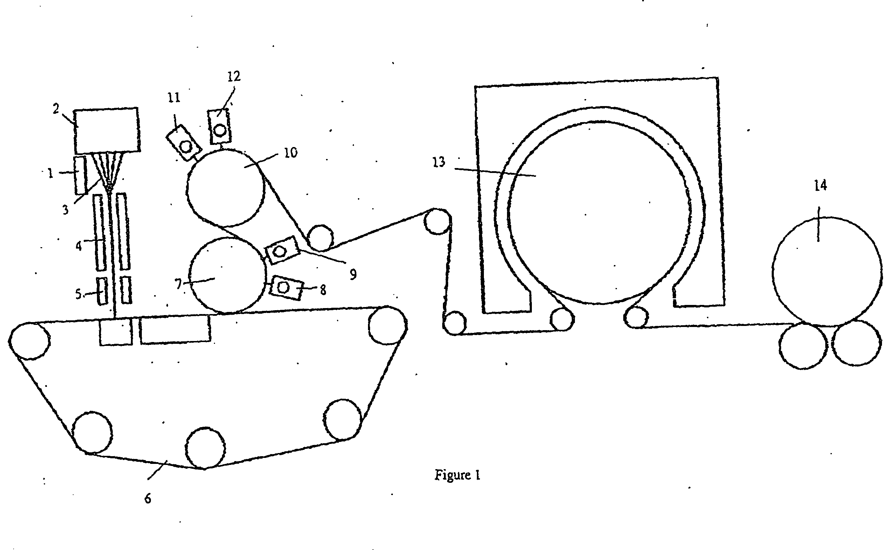

Cette machine schématisée à la figure 1 comprend une tour spunbond 1 comprenant de haut en bas une poutre de filature 2 délivrant un rideau de filaments continus 3 de polymères thermoplastiques. Les filaments sont refroidis par une dispositif de refroidissement 4 et étirés par un dispositif d'étirage 5 puis projetés sur un tapis transporteur 6 en une nappe de filaments continus non consolidée. En aval de la tour spunbond et de préférence tangent au tapis transporteur 6 est disposé un premier cylindre de consolidation par jets d'eau tel que décrit au WO-FR-.03/01101 qui fait partie du présent mémoire. Le premier cylindre 7 est formé d'un tambour comportant un corps cylindrique fixe à surface latérale perforée. Cet ensemble est entouré d'un manchon troué entraîné en rotation par rapport à l'axe du corps cylindrique. Ce corps cylindrique possède des moyens destinés à créer une dépression à l'intérieur du corps. Un manchon est enfilé sur ce cylindre. Ce manchon possède des micro perforations aléatoirement disposées offrant 5% à 20% d'ouverture, d'un diamètre allant de 50 à 500 microns et de préférence compris entre 200 et 300 microns. Il a une épaisseur comprise entre 0,1 et 0,6 mm et de préférence comprise entre 0,2 et 0,4 mm.

Le cylindre 7 est tangent au convoyeur 6. La nappe de filaments continus est transférée au tambour 7 sur lequel elle est consolidée par l'action de deux injecteurs 8 et 9 délivrant des jets d'eau de 120 microns de diamètre à des pression de 30 à 300 bars. La nappe ainsi consolidée est transférée à un second tambour 10 recouvert de manchons prévus pour réaliser des structures en trois dimensions dans les non-tissés et de préférence des perforations. L'action d'ajourage de la nappe de filament est le résultat de l'action des jets d'eau délivrés par les injecteurs 11 et 12 et des supports dont la surface en trois dimensions a été conçue pour générer de petits ajours dans la nappe de filaments. Les manchons sont de différent type.

Ce sont des tissus métalliques ou plastiques dont les fils on un diamètre-allant de 0,4mm à 0,9mm et de préférence entre 0,5 et 0,8mm en chaîne et allant de 0,3 mm à 1mm et de préférence entre 0,4mm et 0,9mm en trame. Ces tissus ont une armure toile, sergé ou chevron. La contexture de ces tissus est comprise entre 5x5 fils par centimètre à 10x10 fils par centimètre. Ces fils ont une section cylindre ou rectangulaire. Le taux de vide de ces tissus est compris entre 20% et 40% et de préférence entre 22% et 30%. La perméabilité à l'air de ces tissus est comprise entre 500 CFM et 1000 CFM.

Il peut aussi s'agir de surfaces gravées par l'action de laser
ou de surfaces perforées sur lesquelles ont été réalisés des dépôts métalliques en relief. Il peut encore s'agir de surfaces en trois dimensions obtenues par attaque chimique de matériaux métalliques. Ces surfaces en relief peuvent être de forme quelconque : ronde, ovale, rectangulaire, hexagonale ou autre. Ces formes possèdent des angles vifs ou arrondis de 10 à 90° suivant la géométrie. Les hauteurs de ces reliefs sont de 1 à 3 mm. Les sections ont des dimensions comprises entre 0,5 et 2,5mm. Ces parties en reliefs sont entourées par des perforations de 0,3 à 0,8mm de diamètre. L'épaisseur de ce réseau de perforations est comprise entre 0,3 et 0,8mm.

Il peut aussi s'agir de tissus métalliques ou plastiques comportant en surface des reliefs durs en polymères. Ces reliefs sont généralement des gouttes de polymères, de forme arrondie, avec des diamètres compris entre 1,4 et 4 mm et de préférence entre 2mm et 3mm. La hauteur de ces déposes est comprise entre 0, 5mm et 1,8mm. La densité de ces reliefs est comprise entre 1 et 20 gouttes par centimètre carré et de préférence entre 5 et 10 gouttes par centimètre carré. Ces gouttes sont intégrées à la surface d'un tissu métallique ou plastique. Les fils de ces tissus ont un diamètre allant de 0,1mm à 0, 4mm et de préférence entre 0,1 5 et 0,3mm en chaîne et allant de 0,2 mm â0,4mm et de préférence entre 0,25mm et 0,35mm en trame. Ces fils ont une armure toile, sergé ou chevron. La contexture de ces tissus est comprise entre 10x10 fils par centimètre à 40x20 fils par centimètre. Ces fils ont une section rectangulaire, cylindre ou hexagonale. Le taux de vide de ces tissus est compris entre 10°% et 30% et de préférence entre 15% et 25%. La perméabilité à l'air de ces tissus est comprise entre 400 CFM et 600 CFM.

L'action de jets d'eau combinée à ces cylindres déplace les filaments jusqu'à former des ajours.

Les injecteurs 11 et 12 délivrent des jets de 120 microns à des pressions de 50 à 300 bars. Des dispositifs d'aspiration par vide d'air non représentés permettent d'évacuer l'excès d'eau des injecteurs par l'intérieur des tambours et aussi de pré sécher le non-tissé avant le transfert au four de séchage par air traversant 13. Le non-tissé est ensuite enroulé par un dispositif d'enroulement 14.

La partie femelle peut être utilisée à la manière habituelle sous la forme d'une bande collée ou autrement fixée à un vêtement et destinée à coopérer avec une bande formant partie mâle. Mais suivant une réalisation particulièrement intéressante, l'invention a également pour objet un vêtement en non-tissé et tout particulièrement une couche-culotte qui se ferme par une fermeture auto-agrippante, caractérisée en ce que la partie femelle de la fermeture est constituée par la face d'endroit d'une partie vêtante soi-même du vêtement qui est l'une des faces d'un non-tissé tel qu'utilisé suivant l'invention.

Une partie vêtante est une partie dont la fonction principale recouvre la nudité directement ou indirectement, la face d'envers étant en contact ave la peau ou avec un sous-vêtement lorsque l'on porte le vêtement.

L'utilisation du non-tissé suivant l'invention, à la fois comme partie vêtante soi-même du vêtement et comme partie femelle de la fermeture auto-agrippante, a des avantages déterminants :
- on n'a plus à coudre, coller ou à autrement fixer une bande formant partie femelle sur une partie du vêtement. Le coût de confection de la couche-culotte s'en trouve diminué considérablement.
- lorsque l'on ferme la couche-culotte, l'élément mâle n'a pas à être ajusté avec précision sur une bande étroite constituant une partie femelle, puisqu'une très grande partie de la face d'endroit du vêtement soi-même est susceptible de constituer la partie femelle. Fermer la couche culotte ou autre vêtement nécessite moins de soin. Si la fermeture échoue en un endroit
où la partie femelle a été abîmée ou trop sollicitée par des ouvertures et des fermetures successives, il suffit de faire coopérer la partie mâle avec un autre endroit de à partie vêtante du vêtement constituant la partie femelle,
- et à ces avantages s'ajoute le fait que la partie femelle est douce au toucher comme il convient pour une partie vêtante.

Suivant un mode de réalisation très préféré, un motif décoratif est apposé sur la face d'envers du non-tissé. Ce motif décoratif est ainsi protégé des influences extérieures et d'une éventuelle détérioration par l'action intempestive de la partie mâle et néanmoins il est bien visible, parce que le non-tissé suivant l'invention est transparent.

Par vêtement, on entend suivant l'invention non seulement les vêtements proprement dits tels que en particulier les couches-culottes ou les produits textiles pour l'incontinence, mais aussi les accessoires, des vêtements et, à titre exceptionnel, lorsqu'un motif décoratif est apposé sur la face d'envers, la fermeture auto-agrippante soi-même. On obtient pour la première fois une fermeture auto-agrippante dont la partie femelle comporte sur la face inactive une décoration en sorte que cette décoration, tout en étant protégée, est visible lorsque l'on sépare la partie mâle de la partie femelle ou est même visible lorsque la partie mâle est apposée sur la partie femelle si l'on prend soin de constituer la partie mâle en une matière transparente.

Notamment dans une couche-culotte la surface d'endroit de la partie vêtante du vêtement a une surface plus de deux fois, de préférence plus de cinq fois, plus grande que la surface de la partie mâle de la fermeture.

La figure 1 est un schéma d'une installation permettant de fabriquer le non-tissé utilisé suivant l'invention, les figures 2 et 3 illustrent respectivement le dispositif de mesure de la force de cisaillement et de la force de pelage,
les figures 4 à 7 sont des vues au microscope avec un grossissement de 20 fois de parties femelles suivant l'invention d'une fermeture auto-agrippante,

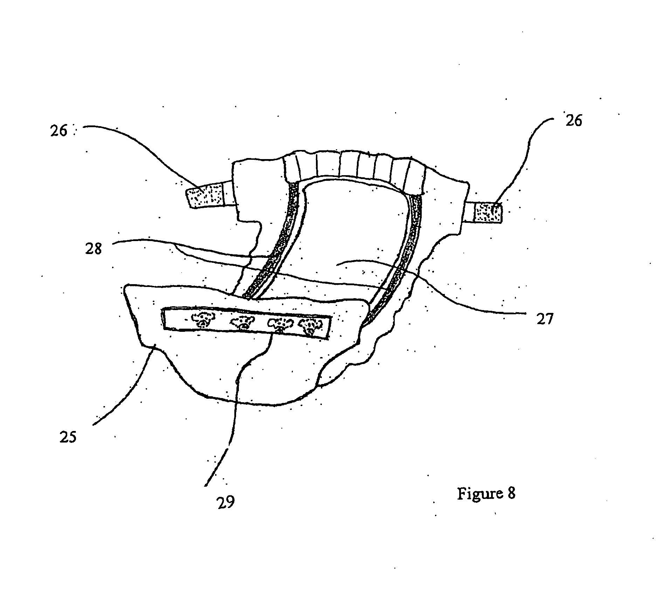

la figure 8 est une vue en perspective d'une couche-culotte suivant l'invention.

Les tests de laboratoire de mesure d'épaisseur, de masse volumique, de résistance dans le sens long et dans le sens travers, d'allongement dans le sens long et dans le sens travers sont conduits selon les normes ERT de l'EDANA (European Disposables And Nonwovens Association), à savoir

### épaisseur :

On conditionne l'échantillon pendant 24 heures et on effectue l'essai à 23°C et à une humidité relative de 50%. On mesure l'épaisseur du non-tissé en mesurant la distance entre un plateau de référence sur lequel repose le non-tissé et un plateau presseur parallèle qui applique une pression précise sur la surface soumise à l'essai. L'appareil consiste en deux plaques horizontales circulaires fixées à bâti. La plaque supérieure se déplace verticalement. Elle a diamètre de 60 mm environ. La plaque de référence a une surface plane d'un diamètre plus grand d'au moins 50 mm que celui de la plaque supérieure.
La pièce d'essais a des dimensions de 100x100 mm à plus ou moins 5 mm. Il est prévu un dispositif de mesure de la distance entre les plaques lorsque celles-ci se sont rapprochées au point d'appliquer une pression de 0.02 kpa sur la pièce d'essai.

### masse au mètre carré:

On conditionne l'échantillon pendant 24 heures et on effectue l'essai à 23°C et à une humidité relative de 50%. On coupe au moins 3 échantillons d'une surface d'au moins 50000 mm2. Chaque échantillon est pesé sur une balance de laboratoire ayant une précision de 0.1% de la masse des échantillons pesés.

### masse volumique:

La masse volumique est calculée à partir de l'épaisseur mesurée et de la masse au mètre carré.
Mv = g/e x 1000
Mv = masse volumique exprimée en grammes par centimètre cube
g = masse au mètre carré du non-tissé exprimée en gramme par mètre carré
e = épaisseur du non-tissé exprimée en millimètres

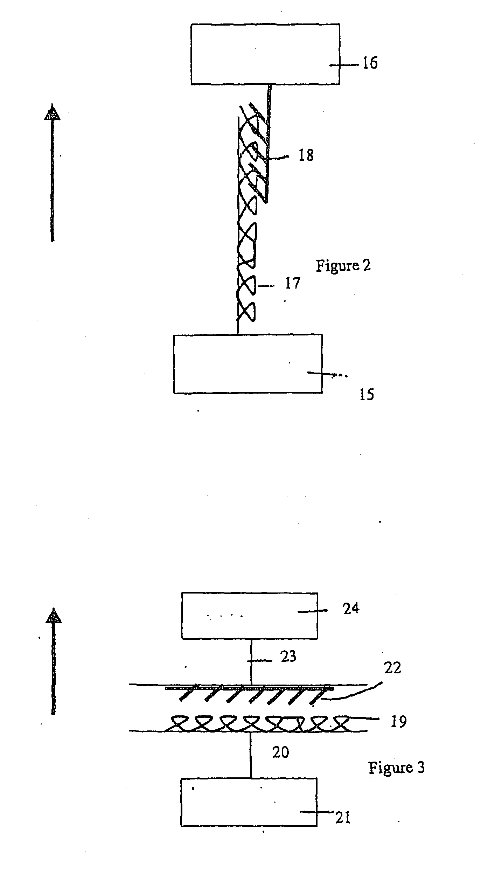

### Force de cisaillement fig 2

On conditionne l'échantillon pendant 24 heures et on effectue l'essai à 23°C et à une humidité relative de 50%. On utilise pour le test un dynamomètre comprenant un jeu de mâchoires fixes 15 et un jeux de mâchoires mobiles 16 se déplaçant à une vitesse constante suivant le sens de la flèche. Les mâchoires du dynamomètre ont une largeur utile de 50 mm. On place le non-tissé 17 sur une plaque cartonnée afin de donner plus de rigidité à cet élément que l'on fixe, ensuite, dans le mors statique 15 du dynamomètre. On bloque le porte-crochet 18 dans le mors mobile 16 après avoir placé la partie crochets (dimension de la zone d'accroche : 25 x 13mm) sur le voile non-tissé 17.
Les crochets utilisés sont du type 963 commercialisés par la société Aplix. Ils comportent 140 crochets au cm2. Les crochets ont une hauteur de 0.36 mm et une largeur de tête de 0.32 mm.

On règle une distance, avant traction; entre les mors de 200mm et une vitesse de traction de 200 mm/min.

L'appareillage utilisé donne la force de traction maximale. Cette dernière correspond à la rupture d'accroche entre ces deux éléments. On procède à 5 essais par échantillon et on calcule la moyenne de 5 essais.

### Force de pelage fig 3

On utilise les même conditions de conditionnement des échantillons et le même dynamomètre que pour le test de cisaillement. Les réglages sont identiques à ceux de l'épreuve de cisaillement. L'échantillon de non-tissé 19 est fixé sur son support en T 20 dans le mors statique 21. Le porte crochets 22 est fixé sur son support en T 23 et fixé dans le mors mobile 24. On procède à 5 essais par échantillon et on calcule la moyenne de 5 essais.

Les exemples suivants et les exemples comparatifs illustrent l'invention.

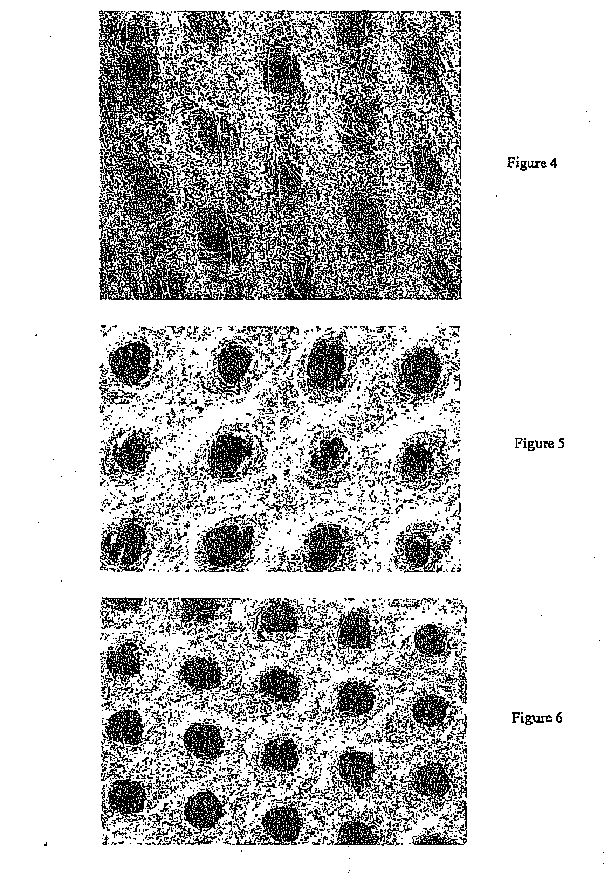

Exemple 1 : On produit un voile de non-tissé de 33 g/m² à une vitesse de 133 mètres par minute avec l'installation conforme à l'invention. Il s'agit d'un non-tissé constitué de filaments continus en PP de 2,2 dtex. Cette nappe de filaments ainsi produite est ensuite consolidée par un premier cylindre. Il est recouvert d'un manchon de nickel micro perforé de trous de 300 microns de diamètre et comporte 100 trous par cm2 répartis de manière aléatoire. Ce premier cylindre comporte deux injecteurs successifs délivrant des jets d'eau de 120 microns de diamètre et avec une densité de jets de 1666 jets par mètre et à des pressions respectivement de 80 bars puis 120 bars. Le voile de filaments ainsi consolidé est transféré au second cylindre. Il est recouvert d'un tissu de bronze de tissage de type toile comprenant 9 fils au cm de section rectangulaire de 0.33 mm x 0,64 mm en sens machine et 9 fils au cm de diamètre 0,46 mm en sens transversal. Le taux de vide du tissu de bronze est de 25% et sa perméabilité à l'air de 875 CFM. Deux injecteurs délivrant des jets d'eau de 120 microns de diamètre et avec une densité de jets de 1666 jets par mètre pour le premier injecteur et de 5000 jets par mètre pour le second agissent à des pressions respectivement de 80 bars et 130 bars sur le voile de filaments pré consolidé. Le non-tissé est ensuite séché dans un four à air traversant à une température de 110°C. Le non-tissé ainsi obtenu présente des ajours de dimensions moyennes de 0,95 x 0,75 mm. La densité de ces ajours est d'environ 25 ajours par cm². fig 4

Exemple 2 On répète l'exemple 1 jusqu'au second cylindre. Le second cylindre est recouvert d'un manchon de tissu métallique fin et non marquant comprenant 22 fils au cm de diamètre 0.20 mm dans le sens machine et 20 fils par cm de diamètre 0.20 mm dans le sens transversal. Ce tissu métallique comporte à sa surface des parties dures en reliefs constituées de polymère et dont le diamètre est de 2.4 mm et dont la hauteur est de 1.25 mm. Il y a 4 parties en relief par cm2. Le non-tissé ainsi obtenu présente des ajours de dimensions moyennes de 2,2 mm et 4 ajours par cm2. fig 5

### Exemple 3 :

On répète l'exemple 1 jusqu'au second cylindre. Le second cylindre est recouvert d'un manchon métallique perforé gravé par enlèvement électrochimique de métal. Il présente des perforations de 0,5 mm de diamètre et des parties en relief de section carrée de 1,5 mm de coté et de hauteur 1,5 mm. Il y a 9 parties en relief par cm2. Le non-tissé ainsi obtenu présente 9 ajours au cm2 de forme carrée et de 1,8 mm de coté. fig 6

### Exemple 4 :

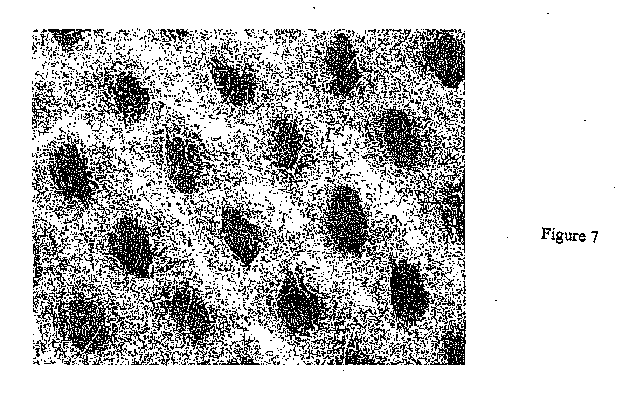

On répète l'exemple 1 jusqu'au second cylindre. Le second cylindre est recouvert d'un manchon métallique perforé gravé par enlèvement électrochimique de métal. Il présente des perforations de 0,5 mm de diamètre et des parties en relief de forme ellipsoïdale de 2,0 mm par 0,75 mm de diamètre et de hauteur 1,5 mm. Il y a 18 parties en relief par cm2. Le non-tissé ainsi obtenu présente 18 ajours au cm2 de forme ellipsoïdale et de diamètre 2,0 par 1,0 mm. fig 7

Comparatif 1 : Un non-tissé commercial de 60 g/m² pour zone d'accroche de couche bébé constitué de filaments de PP de 2,2 dtex. Ce non-tissé décrit dans le brevet US 6,921,570 de Kimberly Clark est utilisé dans les systèmes de fermeture des couches bébé commercialisées sous la marque Huggies. Ce non-tissé calandre négativement, c'est-à-dire que la gravure de la calandre crée sur le voile une consolidation par fusion uniquement entre les formes rondes en relief appelées dots. Ces dots, en relief, sont les points d'accroche des crochets de la partie mâle des systèmes de fermeture.

Comparatif 2 : On répète l'exemple 1 jusqu'au second cylindre. Mais pour cet exemple, le second cylindre est recouvert du même manchons micro perforé que le premier cylindre. Les injecteurs du second cylindre délivrent des jets d'eau de 120 microns de diamètre et avec une densité de jets de 1666 jets par mètre et à des pressions respectivement de 80 bars et 130 bars sur le voile de filaments pré consolidé. Le non-tissé est ensuite séché dans un four à air traversant à une température de 110°C. Le non-tissé ainsi obtenu ne présente pas de perforations et a une surface lisse et uniforme.

| *Tableau récapitulatif* | Exemple 1 | Exemple 2 | Exemple 3 | Exemple 4 | Comparatif 1 | Comparatif 2 |
|---|---|---|---|---|---|---|
| Grammage (g/m²) | 33 | 33 | 33 | 33 | 60 | 33 |
| Epaisseur (mm) | 0.7 | 0.9 | 1.00 | 1.12 | 0.9 | 0.3 |
| Densité (g/cm³) | 0,047 | 0,036 | 0,033 | 0,029 | 0,07 | 0,11 |
| Densités (ajour/cm² | 25 | 4 | 9 | 18 | 0 | 0 |
| Dimensions des ajours (mm) | 0,95 x 0,75 | 2.6 x 2.2 | 1.8 x 1.8 | 2.0 x 1.0 | / | / |
| Force de cisaillement (N) | 16,2 | 15,8 | 23,2 | 17,3 | 13,6 | 13,5 |
| Force de pelage (N) | 18,0 | 16,0 | 17,0 | 20,8 | 10,1 | 14,3 |
| Distance axe médian à axe médian entre 2 ajours (mm) | 0,95 | 2,6 | 3,8 | 2,0 | / | / |

Les non-tissés Utilisés selon l'invention, outre leurs qualités esthétiques, ont plus de voluminosité que les non-tissés du marché employés pour les même applications. Ils ont des performances de force de cisaillement et de force de pelage bien supérieures à celles des non-tissés du commerce utilisés pour les même usages et ceci pour des poids au mètre carré bien inférieur voire réduit de moitié. Ce sont de produits de performance supérieure et de prix compétitif. Ils permettent aussi de réduire le poids de non-tissés employé pour la confection des couches bébé, ce qui constitue l'une des principales attentes du marché actuel.

La couche-culotte suivant l'invention de la figue 8 comporte une partie 25 principale ou vêtante de vêtement en un non-tissé tel qu'utilisé suivant l'invention. A la face extérieure de cette partie principale sont collées deux bandes 26 formant sur leur face intérieure partie mâle d'une fermeture auto-agrippante et destinées à coopérer avec la face d'endroit de la partie 25 pour fermer la couche-culotte. La face d'envers est recouverte dans sa partie d'entrecuisse d'un coeur 27 absorbant en fibres absorbantes et en poudre super-absorbante. Une bande 28 élastique améliore le confort de la couche. On a représenté symboliquement en tirets un motif décoratif 29 qui est apposé sur l'envers de la partie principale 25 et que l'on peut voir par transparence à travers celle-ci.

## Revendications

1. Utilisation, comme partie femelle d'une fermeture auto-agrippante, d'un non-tissé
- qui est constitué de filaments,
- dont la cohésion n'est donnée que par l'enchevêtrement des filaments,
- qui a une épaisseur comprise entre 0,1 et 2,0 mm, de préférence entre 0,25 mm et 1,20 mm,
- qui a un grammage compris entre 10 et 100 g/m2, de préférence entre 15 et 40 g/m2 et
- qui a des ajours en un nombre de 3 à 80 par cm2 , de préférence entre 4 et 40 par cm2, sur les parois latérales desquels les filaments enchevêtrés sont intacts.

2. Utilisation suivant la revendication 1, **caractérisée en ce que** les filaments ont un diamètre compris entre 5 et 50 microns, de préférence entre 10 et 30 microns.

3. Utilisation suivant la revendication 1 ou 2, **caractérisée en ce que** les filaments sont en une matière thermoplastique telle que en polyéthylène, en polypropylène, en poly(téréphtalate d'éthylène), en polyamide, en poly(acide lactique) ou leurs mélanges.

4. Utilisation suivant l'une des revendications précédentes, **caractérisée en ce que** les ajours sont coniques en ayant un angle de cone compris entre 5 et 45°.

5. Utilisation suivant l'une des revendications précédentes, **caractérisée en ce que** les ajours sont disposés de manière ordonnée.

6. Utilisation suivant l'une des revendications précédentes, **caractérisée en ce que** le pas des ajours est compris entre 1,2 et 3 fois et de préférence entre 1,3 et 2,5 fois leur plus petite dimension.

7. Utilisation suivant l'une des revendications précédentes, **caractérisée en ce que** la plus grande dimension des ajours dans le plan du non-tissé est au moins égale à une fois l'épaisseur du non-tissé et au plus égale à 3 fois cette épaisseur.

8. Vêtement en un non-tissé, qui se ferme par une fermeture auto-agrippante, **caractérisé en ce que** la partie femelle de la fermeture est constituée par la face d'endroit d'une partie vêtante soi-même du vêtement, qui est l'une des faces d'un non-tissé tel que défini aux revendications précédentes.

9. Vêtement suivant la revendication 8, **caractérisé par** un motif décoratif apposé sur la face d'envers, qui est l'autre face du non-tissé.

10. Vêtement suivant l'une des revendications 8 ou 9,
**caractérisé en ce que** la face d'endroit a une surface plus de deux fois et, de préférence, plus de cinq fois, plus grande que la surface de la partie mâle de la fermeture.

## Claims

1. Use, as a female part of a hook-and-loop fastener, of a nonwoven
- which is made up of filaments;
- the cohesion of which is provided solely by the entanglement of the filaments;
- which has a thickness of between 0.1 and 2.0 mm, preferably between 0.25 mm and 1.20 mm;
- which has a basis weight of between 10 and 100 g/m², preferably between 15 and 40 g/m²; and
- which has openings numbering between 3 and 80 per cm², preferably between 4 and 40 per cm², on the lateral walls of which the entangled filaments are intact.

2. Use according to claim 1, **characterised in that** the filaments have a diameter of between 5 and 50 microns, preferably between 10 and 30 microns.

3. Use according to claim 1 or 2, **characterised in that** the filaments are made of a thermoplastic material such as polyethylene, polypropylene, poly(ethylene terephthalate), polyamide, poly(lactic acid) or mixtures thereof.

4. Use according to one of the preceding claims,
**characterised in that** the openings are conical, having a cone angle of between 5 and 45°.

5. Use according to one of the preceding claims,
**characterised in that** the openings are disposed in an ordered manner.

6. Use according to one of the preceding claims,
**characterised in that** the pitch of the openings is between 1.2 times and 3 times, and preferably between 1.3 and 2.5 times, their smallest dimension.

7. Use according to one of the preceding claims,
**characterised in that** the largest dimension of the openings in the plane of the nonwoven is at least equal to one times the thickness of the latter and at most equal to 3 times the said thickness.

8. A garment which is made of a nonwoven and is fastened by a hook-and-loop fastener, **characterised in that** the female part of the fastener is constituted by a right-side face of a self-clothing part of the garment, which is one of the faces of a nonwoven as defined in the preceding claims.

9. A garment according to claim 8, **characterised by** a decorative affixed to a reverse-side face, which is the other face of the nonwoven.

10. A garment according to either of claims 8 or 9,
**characterised in that** the right-side face has a surface which is more than two times, and preferably more than five times, larger than the surface of the male part of the fastener.

## Patentansprüche

1. Verwendung eines Faservlieses als Schlaufen umfassendes Teil für einen selbsthaftenden Verschluss,
- das aus Filamenten besteht,
- dessen Zusammenhalt nur durch das gegenseitige Ineinandergreifen der Filamente gegeben ist,
- das eine Dicke zwischen 0,1 und 2,0 mm, vorzugsweise zwischen 0,25 und 1,20 mm hat,
- das ein Flächengewicht zwischen 10 und 100 g/m², vorzugsweise zwischen 15 und 40 g/m² hat, und
- das Durchbrüche in einer Anzahl von 3 bis 80 pro cm², vorzugsweise zwischen 4 und 40 pro cm² hat, an deren Seitenwänden die ineiriandergreifenden Filamente intakt sind.

2. Verwendung nach Anspruch 1, **dadurch gekenzeichnet, dass** die Filamente einen Durchmesser zwischen 5 und 50 Mikrometer, vorzugsweise zwischen 10 und 30 Mikrometer haben.

3. Verwendung nach Anspruch 1 oder 2, **dadurch gekennzeichnet, dass** die Filamente aus einem thermoplastischen Material wie Polyethylen, Polypropylen, Polyethylenterephthalat, Polyamid, Polymilchsäure oder deren Mischungen bestehen.

4. Verwendung nach einem der vorhergehenden Ansprüche, **dadurch gekennzeichnet, dass** die Durchbrüche konisch sind, wobei sie einen Konuswinkel zwischen 5 und 45° haben.

5. Verwendung nach einem der vorhergehenden Ansprüche, **dadurch gekennzeichnet, dass** die Durchbrüche auf geordnete Weise angeordnet sind.

6. Verwendung nach einem der vorhergehenden Ansprüche, **dadurch gekennzeichnet, dass** der Abstand der Durchbrüche das 1,2- bis 3-fache und vorzugsweise das 1,3- bis 2,5-fache ihrer kleinsten Abmessung beträgt.

7. Verwendung nach einem der vorhergehenden Ansprüche, **dadurch gekennzeichnet, dass** die größte Abmessung der Durchbrüche in der Ebene des Faservlieses mindestens gleich der einfachen Dicke des Faservlieses und höchstens gleich dem 3-fachen dieser Dicke ist.

8. Kleidungsstück aus einem Faservlies, das sich durch einen selbsthaftenden Verschluss verschließen lässt, **dadurch gekennzeichnet, dass** das Schlaufen umfassende Teil des Verschlusses durch die Vorderseite eines eigentlichen Bekleidungsteils des Kleidungsstücks gebildet ist, die eine der Seiten eines wie in den vorhergehenden Ansprüchen definierten Faservlieses ist.

9. Kleidungsstück nach Anspruch 8, **gekennzeichnet durch** ein an der Rückseite, welche die andere Seite des Faservlieses ist, angebrachtes Dekor.

10. Kleidungsstück nach einem der Ansprüche 8 oder 9, **dadurch gekennzeichnet, dass** die Vorderseite eine Oberfläche hat, die mehr als zwei Mal und vorzugsweise mehr als fünf Mal größer ist als die Oberfläche des Häkchen umfassenden Teils des Verschlusses.
